# EUROPEAN PATENT SPECIFICATION

(11) **EP 4 188 289 B1**
(45) Date of publication and mention of the grant of the patent: **10.04.2024**
(21) Application number: 21758279.0
(22) Date of filing: 27.07.2021
(51) Int. Cl.: A61F 5/44, A61F 5/445

(54) **SYSTEM AND METHOD FOR OSTOMY INFORMATION COLLECTION AND ANALYSIS**
SYSTEM UND VERFAHREN ZUR SAMMLUNG UND ANALYSE VON STOMAINFORMATIONEN
SYSTÈME ET MÉTHODE DE COLLECTE ET D'ANALYSE D'INFORMATIONS SUR LES STOMIES

(30) Priority: 29.07.2020 US 202063058214 P
(43) Date of publication of application: 07.06.2023
(60) Divisional application: 24154103.6
(73) Proprietor: Hollister Incorporated, Libertyville, IL 60048-3781 (US)
(72) Inventor: CARLSSON, Jonas, P., Libertyville, IL 60048 (US); PARK, Ryan, S., Libertyville, IL 60048 (US)
(74) Representative: FRKelly
(86) International application number: PCT/US2021/070961
(87) International publication number: WO 2022/026995

(56) References cited:
- WO-A1-2019/120458
- WO-A1-2019/169327
- WO-A1-2020/076607
- WO-A1-2020/076609
- WO-A2-2020/076605
- US-A1- 2019 133 811
- US-A1- 2019 240 059
- US-A1- 2020 000 624

## Description

### BACKGROUND

The following description relates generally to an ostomy leakage detection system, and in particular, to a system and method for ostomy information collection and analysis.

An ostomy pouch system typically includes a pouch formed from opposing sidewalls defining an internal collection area, an inlet opening for receiving a stoma, and an ostomy appliance for attaching the pouch to a user. The ostomy appliance may include, for example, an ostomy barrier of a one-piece pouch system, which is attached to one of the pouch sidewalls proximate an inlet opening, a faceplate for a two-piece pouch system configured to releasably engage a pouch, and a barrier ring. The ostomy appliance may include a skin barrier material for adhering to and sealing against user's peristomal skin surrounding the stoma.

The ostomy appliance may be susceptible to ostomy effluent leakage, and the seal formed between the skin barrier material and the user may weaken. Often times, the user may be unaware of or cannot easily assess an extent of weakening in the seal. Thus, the user may not become aware of a weakened seal, and consequently, the ostomy effluent may leak through to an exterior of the ostomy appliance.

US2019133811A1 discloses an ostomy bag can including one or more sensors for measuring one or more metrics. An ostomy wafer can also include one or more sensors for measuring one or more metrics. The sensors can be temperature sensors and/or capacitive sensors, for example, and the metrics can include bag fill, leakage, skin irritation, and phase of stoma output, among others.

WO2019120458A1 discloses a base plate for an ostomy appliance, the base plate (4) comprises a top layer (208) defining a base plate plane; a first adhesive layer (200) adapted to adhere the base plate (4) to peristomal skin of a user; an electrode assembly (204); and a monitor interface (207) configured to electronically connect with the electrode assembly (204), where the monitor interface (207) comprises a coupling part (210) configured to form a releasably mechanically and/or electronically coupling between the base plate (4) and a monitor device (6); the coupling part (210) is configured to engage and/or disengage with the monitor device (6) allowing the monitor device (6) to be coupled to the base plate by a motion in a direction corresponding to an acute angle of 45 degrees or less relative to the base plate plane. WO2019120458A1 also discloses the preamble of claim 1.

US2019240059A1 discloses systems and methods of providing post-operative ostomy patient care which can include software and/or hardware that facilitates patient care personnel, such as a patient coach, providing remote patient care or coaching over a network to an ostomy patient. The care may be provided by specially trained former patients, nurses, or other care providers. The system can integrate sensor data from an ostomy device and inputs from the former patients, healthcare professionals, and/or device manufacturer in provide the post-operative care.

WO2019169327A1 discloses a method of detecting a fill level of an ostomy bag of a draining device can be based on resistance readings from a resistance sensor mounted on the ostomy bag and/or a user input that the ostomy bag has been drained. Smoothing algorithms can be implemented to identify drain data points by filtering out noises in a fill volume signal obtained based on the resistance readings.

US2020000624A1 discloses an ostomy leakage alert system for notifying a user of ostomy leakage which includes a sensor assembly comprising a sensor pad and a sensor integrally disposed within the sensor pad. The sensor pad has an aperture to couple with an ostomy bag and an ostomy skin barrier disc. A lead has a sensor end coupled to the sensor and a connector end having a male connector. An alarm assembly is coupled to the lead, the alarm assembly comprising an alarm housing, a battery, a CPU, an alarm speaker, a female port, and an N-channel. The female port selectively receives the male connector of the lead. The battery, the CPU, the alarm speaker, the female port, and the N-channel are disposed within the alarm housing. The sensor assembly is configured to detect a leakage from the ostomy bag and to activate the alarm assembly, notifying a wearer of the leakage.

WO2020076605A2 discloses an ostomy appliance includes an ostomy hydrocolloid having a body-facing side, a pouch-facing side and a stoma opening, at least one fluid aperture formed in the body-facing side and at least one fluidic channel embedded in the ostomy hydrocolloid having a proximal portion disposed in fluid communication with the at least one fluid aperture. The ostomy appliance also includes at least one window formed in the pouch facing side, the at least one window aligned with a distal portion of the fluidic channel such that a portion of the fluidic channel is visible through the window. In another embodiment, an ostomy appliance includes a skin barrier material and an ultrasonic detection device for monitoring the skin barrier material.

WO2020076609A1 discloses an ostomy appliance system includes an ostomy appliance having a stoma opening and a first electrical interface, and at least one thermal sensor configured to detect at least one thermal property, such as temperature, at the ostomy appliance, the at least one temperature sensor connected to the first electrical interface with electrical circuitry. A wearable device may be removably connected to the ostomy appliance and operably connected to the at least one thermal sensor. The wearable device includes a housing, a second electrical interface configured for electrical connection to the first electrical interface, a power supply and a controller operably connected to the power supply. The controller is configured to determine a condition of the ostomy appliance based on the at least one thermal property detected at the at least one thermal sensor. The wearable device may also include a wireless transceiver configured to communicate with a personal communication device.

WO2020076607A1 discloses an ostomy appliance includes a substrate and at least one Radio Frequency Identification (RFID) circuit disposed on the substrate. The RFID circuit includes a RFID transponder having an antenna and a conductive ink connected in series with the antenna and the RFID transponder. The conductive ink is configured to dissolve in response to exposure to moisture. The RFID circuit is in a closed condition when the conductive ink extends continuously between the RFID transponder and the antenna. The RFID circuit is in an open condition when at least a portion of the conductive ink is dissolved.

Accordingly, it is desirable to provide a leakage detection system for ostomy products and a system and method for ostomy information collection and analysis.

### SUMMARY

In one aspect, an ostomy leak detection system includes a sensor device configured to detect one or more parameters or parameter values indicative of a condition of an ostomy product, and one or more auxiliary devices operably connected to the sensor device and configured to receive sensor information indicative of the condition of the ostomy product from the sensor device. The one or more auxiliary devices includes at least one of monitoring device, a wearable device, a consumer electronic device and a server device.

The ostomy leak detection system may be configured to detect, determine and/or receive ostomy system information and store the ostomy system information. The ostomy leak detection system may determine characteristic information based at least in part on the ostomy system information. According to the invention, the ostomy leak detection system may aggregate and store ostomy system information and/or characteristic information from a plurality of users and/or ostomy products.

In an embodiment, the sensor device may be a leakage sensor device configured to detect electrical resistance. In an embodiment, the one or more auxiliary devices may include the wearable device operably connected to the sensor device and a consumer electronic device communicably connected to the wearable device via a wireless connection. In an embodiment, the one or more auxiliary devices may include a monitoring device operably coupled to the sensor device, a wearable device communicably coupled to the monitoring device, and a consumer electronic device communicably connected to the wearable device via a wireless connection.

In an embodiment, the at least one auxiliary device may include a server device communicably coupled to one or more of the wearable device and the consumer electronic device. In an embodiment, the ostomy leak detection system may further include an ostomy product. The sensor device may be operably connected to the ostomy product.

In another aspect, a method for ostomy leak detection system analysis includes receiving ostomy system information relating to an ostomy product. The ostomy system information may be information relating to factors affecting performance of the ostomy product. The method also includes determining characteristic information based on the ostomy system information.

In an embodiment, the method may further include providing a notification based on the characteristic information. According to the invention, receiving ostomy system information may further include aggregating ostomy system information from multiple ostomy leak detection systems. In an embodiment, the characteristic information may include at least one of product consumption information, leak information, inventory information, stoma health information, replacement information, recommendation information and predictive information.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a block diagram schematically illustrating an example of an ostomy leak detection system according to an embodiment;
FIG. 2 is a block diagram schematically illustrating an example of an auxiliary device according to an embodiment;
FIG. 3 is a diagram illustrating an example of a device memory according to an embodiment; and
FIG. 4 is a block diagram illustrating an example of a method for ostomy leak detection analysis according to an embodiment.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

According to embodiments, an ostomy leak detection system may generally include a sensor device and one or more auxiliary devices. The sensor device may be used with an ostomy product, such as an ostomy appliance, ostomy pouch or ostomy accessory. The ostomy appliance may be, for example, an ostomy barrier or faceplate. The ostomy pouch may be coupled to the ostomy appliance. The ostomy appliance may be adhered to a user's body to seal around a stoma and the ostomy pouch may receive effluent discharged from the stoma. In an embodiment, the ostomy accessory may be attached to a body side of the ostomy appliance and thus, may be disposed between the user and the ostomy appliance when secured to the user. In an embodiment, the ostomy leak detection system may include one or more ostomy products.

The ostomy leak detection system may be configured to receive information from one or more sources, such as, but not limited to, a sensor device, an auxiliary device and/or user input. The ostomy leak detection system may also determine information, for example, based on the received information. In an embodiment, the received information may be ostomy system information. Ostomy system information may be information associated with various factors which may affect performance of an ostomy product. Information determined by the ostomy leak detection system based at least in part on the ostomy system information may be referred to generally herein as characteristic information. Characteristic information may refer to information relating performance characteristics of an ostomy product.

In an embodiment, the sensor device may be configured to provide sensor information to one or more auxiliary devices. For example, the sensor device may be configured to detect one or more parameters or parameter values which may be indicative of a condition or performance characteristic of an ostomy product. Such parameters may include, for example, electrical resistance, impedance, color, temperature, strain, light and the like. In an embodiment, the sensor device may be a leakage sensor configured to provide sensor information for determining whether effluent is leaking radially outward from a stoma opening. For example, the sensor device may detect values of one or more parameters which may be indicative of a leak, such as, but not limited to, electrical resistance. The sensor device may detect the one or more parameters at a surface of or within the ostomy appliance or ostomy accessory. In an embodiment, the sensor device may be at least partially disposed on a body side of the ostomy appliance or ostomy accessory and may be configured to detect the one or more parameters at the user's skin surface.

The one or more auxiliary devices may include, for example, a monitoring device, a wearable device, a consumer electronic device, and/or a server device. One or more of the auxiliary devices may include a processor, a memory and a communication device. The processor may be, for example, a microprocessor or other suitable computer processor or processor-like device. The processor may be configured to execute program instructions and perform operations based on the executed program instructions. For example, the processor may control operations of other devices or components based on the executed program instructions. The memory may be a non-transitory computer-readable storage medium configured to store the program instructions. The memory may also store other information, such as the sensor information provided by the sensor device. Sensor information may include, for example, parameter information and/or parameter value information. The communication device may be a transceiver or transceiver-like device. The communication device may be configured to transmit and/or receive information via a wired and/or wireless communication interface.

One or more of the auxiliary devices may also include a user interface device. The user interface device may be configured to receive input information from a user and/or may be configured to output information to a user. The user interface device may include, for example, one or more of a keyboard, key pad, pointer, button, switch, knob, lever, dial, touchscreen display, microphone, speaker, haptic motor, lights or LEDs, biometric sensor, optical sensor and/or combinations thereof. Other known, suitable user interfaces are envisioned as well.

In some embodiments, the sensor device may be integrated or combined with one more of the auxiliary devices and may include one or more of the components of the auxiliary devices described above. For example, in an embodiment, the sensor device and the monitoring device may be provided together as a unit.

In an embodiment, the sensor device and the monitoring device may be provided at the ostomy appliance. The monitoring device may receive the sensor information from the sensor device. The wearable device may be removably coupled to monitoring device and the ostomy appliance. The wearable device may be communicably connected to the monitoring device to receive information, such as the sensor information, from the monitoring device. In an embodiment, the wearable device may be communicably connected via a wireless connection to, for example, a consumer electronic device, such as a smart phone, tablet computer, laptop computer, desktop computer and the like. The consumer electronic device may receive information from the wearable device, such as the sensor information. The consumer electronic device may also transmit information to the wearable device. The consumer electronic device may be communicably connected to a server device, for example, over a communication network such as the Internet and/or a cellular network, and may transmit information to, and/or receive information from, the server device. In other embodiments, the wearable device may be communicably connected to the consumer electronic device via a wired connection and may transmit and/or receive information via the wired connection.

In other embodiments, the monitoring device may be communicably connected to the wearable device via a wireless connection. In still other embodiments, the monitoring device may be omitted, or integrated with the wearable device, and the wearable device may be connected directly to the sensor device and may receive information, such as sensor information, from the sensor device.

In some embodiments, the sensor device may be synced or baselined with one or more of the auxiliary devices to facilitate communication between the sensor device and the one or more auxiliary devices. The sensor device may be synced or baselined each time an ostomy product, such as an ostomy appliance, ostomy accessory or ostomy pouch is changed out or replaced. Accordingly, the auxiliary device may count a number of syncing operations and save the count as product consumption information. The syncing or baselining operation may be product specific. Accordingly, product consumption may be tracked based on the stored product consumption information. Use information may be tracked and stored as well. Use information may include, for example, length of use of an ostomy product. The use information may be derived from the product consumption information, for example, based on a length of time between syncing/baselining operations for a particular ostomy product.

An auxiliary device of the ostomy leak detection system may also determine inventory information for an ostomy product. The inventory information may be based at least in part on the product consumption information. In addition, SKU information of an ostomy product may be received by an auxiliary device. The SKU information may include, for example, quantity information of the ostomy product. The auxiliary device may further determine inventory information based on the quantity information.

The ostomy leak detection system, for example, by an auxiliary device, may provide a notification regarding the inventory information. For example, the notification may alert a user to a number of remaining ostomy products in a given supply. In an embodiment, the notification may be provided when the number of remaining ostomy products falls below a threshold value. In addition, the ostomy leak detection system, for example at an auxiliary device, may provide a prompt to a user to re-order an ostomy product based on the inventory information. The auxiliary device may be communicably connected to an ostomy product supplier and may re-order the ostomy product based on the inventory information.

In an embodiment, the ostomy leak detection system may be configured to detect, determine, or receive additional information. Additional information may be received, for example, via user input, sensors or other devices. The additional information may include, for example, leak information, gas information, and/or diet information. The additional information may be stored at one or more of the auxiliary devices.

In an embodiment, the ostomy leak detection system may be configured to detect, determine and/or receive stoma health information (including skin health information). In an embodiment, images of the stoma and/or peristomal skin may be captured by an image capture device. The images may be photographs, thermal images, or other suitable images. The images may be captured as a function of time. The images may be stored, for example, in the memory of an auxiliary device. The image capture device may be, for example, a camera, and may be integrated with or connected to an auxiliary device, such as a smart phone camera. The stoma health information may be determined based on an image or series of images which may show a change in appearance over time. In an embodiment, images of the stoma and/or peristomal area may be taken before application of an ostomy appliance to the user and upon removal of the ostomy appliance from the user.

In an embodiment, the ostomy leak detection system may be configured to contact an ostomy specialist, such as a WOC/ET nurse. Other ostomy specialists including technical support personnel, customer service personnel and the like may be contacted as well. In an embodiment, information, including images, stoma health information or other relevant information, stored in the ostomy leak detection system may be shared with or accessed by the ostomy specialist. In an embodiment, the ostomy specialist may be contacted via a consumer electronic device, such as a smart phone, tablet computer, laptop computer, desktop computer or other device configured for communication over a communication network. The ostomy specialist may be able to provide information to the user via the ostomy leak detection system, such as troubleshooting information or additional stoma health information including diagnostic information.

The stoma health information, including skin health information, may indicate a change in stoma health over time. Thus, the stoma health information may be beneficial from a clinical and/or health economic perspective.

The ostomy leak detection system, for example, by an auxiliary device, may be configured to detect, determine and/or receive wear time information for an ostomy product. In an embodiment, wear time information may be determined by aggregating information which may be relevant to product wear time from multiple users and analyzing the aggregated information. The aggregated information may include, for example, user country information, ostomy type information, and product type information, including long versus short wear information. The aggregated wear time information may be analyzed to determine, for example, average or median wear time for different products, in different locations, and for different ostomy types. Additional wear time information may be aggregated as well, including, but not limited to user age information, climate information, user activity information, product age information, product manufacture information, product source information and the like.

The aggregated wear time information may be useful for market access reasons, for example, to change utilization rates, change reimbursement rates, change standard practice in terms of recommended wear time per ostomy product.

In an embodiment, the ostomy leak detection system, for example, by an auxiliary device, may be configured to determine predictive information based on determined, detected and/or received information. In an embodiment, the predictive information may further be based on an artificial intelligence or machine learning training model and training data. In this manner, the ostomy leak detection system may track leaks and understand trends to determine predictive information for approximating future leak patterns and trends for particular products, user or both. The ostomy leak detection system may provide such information to a user, ostomy specialists, ostomy suppliers, ostomy manufacturers and the like.

In an embodiment, the predictive information may be based, at least in part, on sensor information provided by the sensor device. The sensor information may be, for example, information indicative of an effluent leak, an extent of effluent, and/or a location of an effluent leak. The predictive information may be based on other information as well, such as product information (e.g., type, model, etc.), ostomy type information, environmental information, activity information, wear time information, and/or other information relevant for predicting performance of an ostomy product. Aggregated data may be used as well.

By learning a patient and their leakage trends, the ostomy leak detection system may be able to predict when the ostomy product worn by the patient may be expected to leak and may alert the patient to change the ostomy product according to the predictive information. This may also be useful for providing product recommendations, e.g., to use a convex ostomy product. For example, if over 1 year, the patient typically experiences leakage at 9 o'clock after 2 days of use, the predictive information may be determined and could be used along with a clinician consult/recommendation to change the product they are using.

Different end users (i.e., patients) may have different types of stomas, outputs, body types, ages and the like. Accordingly, ostomy leak detection systems may store different user information depending on the end user, including but not limited to stoma type information, output information, body type information, age information and the like.

In an embodiment, the user information from different users may be aggregated. Similar attributes or information across the different users may be identified and used to provide, for example, product recommendations for the clinician and/or end user and may be able to influence which product(s) end user should use in certain timelines, for example, a first ostomy product may be recommended for a particular user post operation, a second product after 6 months, and a third product after 1 year. The present description is not limited to these examples however, and it is appreciated that a different number of products and different timelines may be recommended.

In addition, clinicians may limit inventory based on the predictive information by stocking products which are recommended for use for particular patients based on the predictive information. It is also envisioned that information detected, determined and/or received by the ostomy leak detection system may be used by an ostomy manufacturer or designer to design new ostomy products.

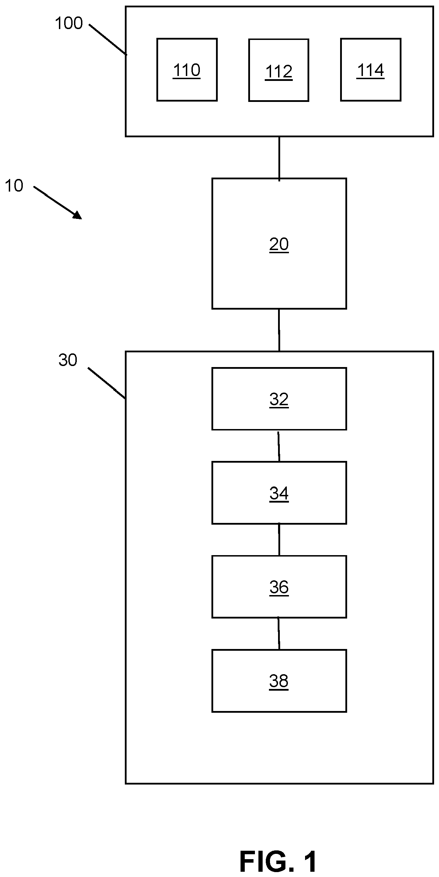

FIG. 1 is a block diagram schematically illustrating an example of an ostomy leak detection system 10 according to embodiments. The ostomy leak detection system 10 may include, for example, a sensor device 20 and one or more auxiliary devices 30. The one or more auxiliary devices 30 may include, for example, a monitoring device 32, a wearable device 34, a consumer electronic device 36 and/or a server device 38. In an embodiment, the ostomy leak detection system 10 may include or be coupled with one or more ostomy products 100, such as an ostomy appliance 110, an ostomy accessory 112 and/or an ostomy pouch 114.

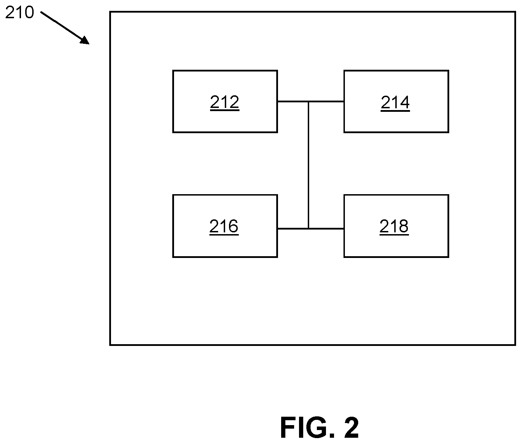

FIG. 2 is a block diagram schematically illustrating an example of an auxiliary device 210 according to an embodiment. The auxiliary device 210 may include, for example, a processor 212, a memory 214, a communication device 216, and a user interface device 218.

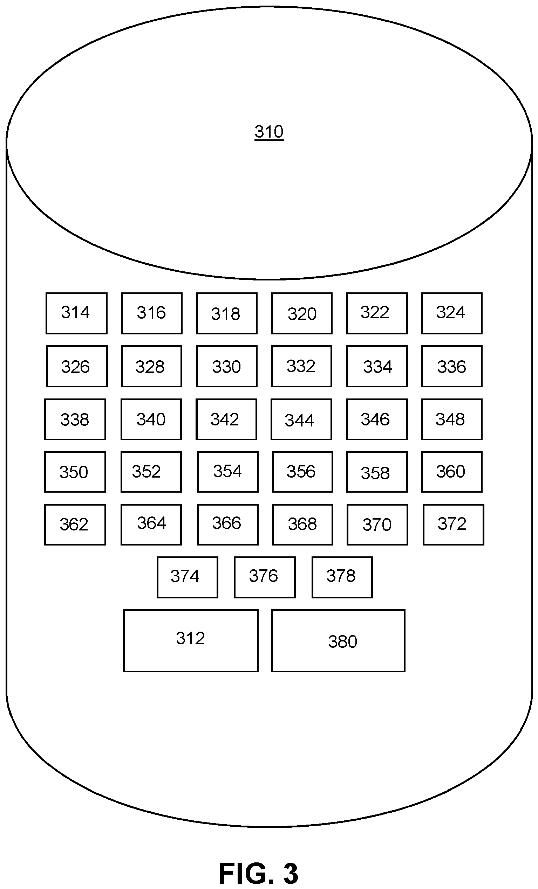

FIG. 3 is a diagram schematically illustrating an example of a memory 310 according to an embodiment. The memory 310 may be configured to store, for example, program instructions 312. The memory may also be configured to store various information detected, determined or received by the ostomy leak detection system such as the ostomy system information and/or characteristic information. For example, the stored information may include, but is not limited to, sensor information 314, parameter information 316, product consumption information 318, use information 320, inventory information 322, SKU information 324, quantity information 326, additional information 328, leak information 330, gas information 332, diet information 334, stoma health information 336, images or image information 338, troubleshooting information 340, diagnostic information342, wear time information 344, aggregated information 346, user country and/or location information 348, ostomy type information 350, product type information 352, user age information 354, climate information 356, user activity information 358, product age information 360, product manufacture information 362, product source information 364, predictive information 368, environmental information 370, stoma type information 372, stoma output information 374, body type information 376, recommendation information 378, training model(s) 380 and the like. The memory 310 may comprise a plurality of memory devices at different auxiliary devices. Each of the memory devices may be configured to store some or all of the information of the present embodiments. Memory devices of different auxiliary devices may store different information from one another as well.

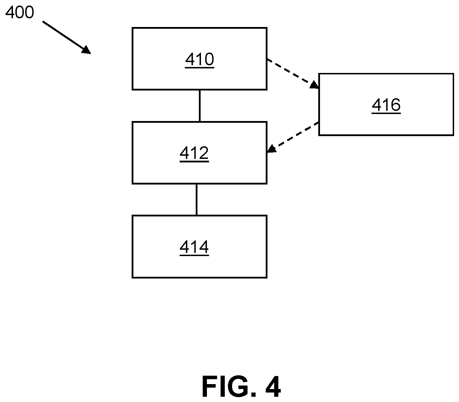

FIG. 4 is a block diagram illustrating a method 400 for ostomy leak detection system analysis according to an embodiment. At 410, the method may include receiving ostomy system information relating an ostomy product. The ostomy system information may be received, for example, via a sensor device (e.g., detected information), user input, and/or from an auxiliary device including information stored by an auxiliary device or determined by an auxiliary device. The ostomy system information may include, for example, any information described in embodiments herein and/or any other relevant information relating to factors which may affect performance of an ostomy product. In an embodiment, the ostomy system information may be received by an auxiliary device.

At 412, the method may include determining characteristic information based on the ostomy system information. Characteristic information may include, for example, product consumption information, leak information, inventory information, stoma health information, replacement or time to replace information, recommendation information, predictive information and the like. One or more of the auxiliary devices may determine the characteristic information.

At 414, the method may optionally include providing a notification. The notification may be provided based on, for example, the determined characteristic information. The notification may be provided by one or more of the auxiliary devices.

At 416, the method may optionally include aggregating ostomy system information from a plurality of ostomy leak detection systems corresponding to a plurality of different users and/or ostomy products. Characteristic information may be determined based on the aggregated ostomy system information. One or more of the auxiliary devices may be configured to aggregate the ostomy system information. In an embodiment, the characteristic information may be aggregated as well, for example, by one or more of the auxiliary devices. For example, in an embodiment, a server device may aggregate ostomy system information and/or characteristic information.

It will be appreciated that numerous factors not specifically described above may affect performance of an ostomy product, such as an ostomy accessory, an ostomy appliance and/or an ostomy pouch. Such factors may be specific to the user, the ostomy product, the environment, and/or the manufacturer, for example. Information relating to such factors, such as the ostomy system information, may be provided to an ostomy leak detection system and used as a basis for determining (including predicting) one or more characteristics of the ostomy product. The factors affecting performance of an ostomy product and the one or more characteristics of the ostomy product may further be used by ostomy specialists, clinicians, users and other relevant personnel for managing ostomy care for the patient/user.

In the present disclosure, the words "a" or "an" are to be taken to include both the singular and the plural. Conversely, any reference to plural items shall, where appropriate, include the singular. In additions, various features described with respect to any of the embodiments above may be used together, implemented in, or replace features in any of the other embodiments described above.

From the foregoing it will be observed that numerous modifications and variations can be effectuated without departing from the scope of the novel concepts of the present invention. It is to be understood that no limitation with respect to the specific embodiments illustrated is intended or should be inferred. The disclosure is intended to cover by the appended claims all such modifications as fall within the scope of the claims.

## Claims

1. An ostomy leak detection system (10) comprising:
a sensor device (20) configured to detect one or more parameters or parameter values indicative of a condition of an ostomy product; and
one or more auxiliary devices (30) operably connected to the sensor device and configured to receive sensor information indicative of the condition of the ostomy product from the sensor device,
wherein the one or more auxiliary devices includes at least one of monitoring device (32), a wearable device (34), a consumer electronic device (36) and a server device (38),
wherein the one or more of the auxiliary devices is configured to determine and/or receive an ostomy system information, wherein the one or more auxiliary devices are configured to determine characteristic information of an ostomy product based on the ostomy system information, and **characterised in that**
the one or more auxiliary devices are configured to aggregate and store the ostomy system information and/or the characteristic information from a plurality of users and/or ostomy products.

2. The ostomy leak detection system (10) of claim 1, wherein the sensor device (20) is a leakage sensor device configured to detect electrical resistance.

3. The ostomy leak detection system (10) of any of claims 1-2, wherein the one or more auxiliary devices (30) includes the wearable device (34) operably connected to the sensor device and a consumer electronic device (36) communicably connected to the wearable device via a wireless connection.

4. The ostomy leak detection system (10) of any of claims 1-2, wherein the one or more auxiliary devices (30) includes a monitoring device (32) operably coupled to the sensor device (20), a wearable device (34) communicably coupled to the monitoring device, and a consumer electronic device (36) communicably connected to the wearable device via a wireless connection.

5. The ostomy leak detection system (10) of any of claims 1-4, wherein the at least one auxiliary device (30) includes a server device (38) communicably coupled to one or more of the wearable device (34) and the consumer electronic device (36).

6. The ostomy leak detection system (10) of any of claims 1-5, further comprising an ostomy product (100), wherein the sensor device (20) is operably connected to the ostomy product (100).

7. A method (400) for ostomy leak detection system analysis comprising:
receiving ostomy system information relating to an ostomy product, wherein the ostomy system information is information relating to factors affecting performance of the ostomy product;
determining characteristic information based on the ostomy system information; and
**characterised in that** the method comprises aggregating and storing the ostomy system information and/or the characteristic information from a plurality of users and/or ostomy products.

8. The method (400) of claim 7, further comprising providing a notification based on the characteristic information.

9. The method (400) of any of claims 7-8, wherein the characteristic information includes at least one of product consumption information, leak information, inventory information, stoma health information, replacement information, recommendation information and predictive information.

## Patentansprüche

1. Ostomie-Leckerkennungssystem (10), umfassend:
eine Sensorvorrichtung (20), die zum Erfassen eines oder mehrerer Parameter oder Parameterwerte, die einen Zustand eines Ostomieprodukts anzeigen, konfiguriert ist; und
eine oder mehrere Hilfsvorrichtungen (30), die mit der Sensorvorrichtung wirkverbunden und zum Empfangen von Sensorinformationen, die den Zustand des Stomaprodukts anzeigen, von der Sensorvorrichtung konfiguriert sind,
wobei die eine oder die mehreren Hilfsvorrichtungen mindestens eines von Überwachungsvorrichtung (32), tragbarer Vorrichtung (34), Unterhaltungselektronikvorrichtung (36) und Servervorrichtung (38) beinhalten,
wobei die eine oder mehreren der Hilfsvorrichtungen zum Bestimmen und/oder Empfangen einer Ostomiesysteminformation konfiguriert sind, wobei die eine oder die mehreren Hilfseinrichtungen zum Bestimmen von charakteristischen Informationen eines Ostomieprodukts basierend auf der Ostomiesysteminformation konfiguriert und **dadurch gekennzeichnet sind, dass**
die eine oder die mehreren Hilfsvorrichtungen zum Aggregieren und Speichern der Ostomiesysteminformation und/oder der charakteristischen Informationen von einer Vielzahl von Benutzern und/oder Ostomieprodukten konfiguriert sind.

2. Ostomie-Leckerkennungssystem (10) nach Anspruch 1, wobei die Sensorvorrichtung (20) eine Leckagesensorvorrichtung ist, die zum Erfassen von elektrischem Widerstand konfiguriert ist.

3. Ostomie-Leckerkennungssystem (10) nach einem der Ansprüche 1-2, wobei die eine oder die mehreren Hilfseinrichtungen (30) die tragbare Vorrichtung (34), die mit der Sensorvorrichtung funktional verbunden ist, und eine Unterhaltungselektronikvorrichtung (36), die mit der tragbaren Vorrichtung über eine drahtlose Verbindung kommunikativ verbunden ist, beinhalten.

4. Ostomie-Leckerkennungssystem (10) nach einem der Ansprüche 1-2, wobei die eine oder die mehreren Hilfsvorrichtungen (30) eine Überwachungsvorrichtung (32), die mit der Sensorvorrichtung (20) funktional gekoppelt ist, eine tragbare Vorrichtung (34), die mit der Überwachungsvorrichtung kommunikativ gekoppelt ist, und eine Unterhaltungselektronikvorrichtung (36), die mit der tragbaren Vorrichtung über eine drahtlose Verbindung kommunikativ verbunden ist, beinhalten.

5. Ostomie-Leckerkennungssystem (10) nach einem der Ansprüche 1-4, wobei die mindestens eine Hilfsvorrichtung (30) eine Servervorrichtung (38) beinhaltet, die mit einer oder mehreren der tragbaren Vorrichtung (34) und der Unterhaltungselektronikvorrichtung (36) kommunizierbar gekoppelt ist.

6. Ostomie-Leckerkennungssystem (10) nach einem der Ansprüche 1-5, ferner umfassend ein Ostomieprodukt (100), wobei die Sensorvorrichtung (20) mit dem Ostomieprodukt (100) in Wirkverbindung steht.

7. Verfahren (400) zur Ostomie-Leckerkennungssystemanalyse, umfassend:
Empfangen der Ostomiesysteminformation in Bezug auf ein Ostomieprodukt, wobei die Ostomiesysteminformation Informationen in Bezug auf Faktoren darstellt, die die Leistung des Ostomieprodukts beeinflussen;
Bestimmen von charakteristischen Informationen basierend auf der Ostomiesysteminformation; und
**dadurch gekennzeichnet, dass** das Verfahren Aggregieren und Speichern der Ostomiesysteminformation und/oder der charakteristischen Informationen von mehreren Benutzern und/oder Ostomieprodukten umfasst.

8. Verfahren (400) nach Anspruch 7, ferner umfassend Bereitstellen einer Benachrichtigung basierend auf den charakteristischen Informationen.

9. Verfahren (400) nach einem der Ansprüche 7-8, wobei die charakteristischen Informationen mindestens eines von Produktverbrauchsinformationen, Leckinformationen, Bestandsinformationen, Ostomiezustandsinformationen, Ersatzinformationen, Empfehlungsinformationen und Vorhersageinformationen beinhalten.

## Revendications

1. Système (10) de détection de fuites de stomie, comprenant :
un dispositif de capteur (20) configuré pour détecter un ou plusieurs paramètres ou valeurs de paramètres indiquant un état d'un produit de stomie ; et
un ou plusieurs dispositifs auxiliaires (30) connectés de manière fonctionnelle au dispositif de capteur et configurés pour recevoir des informations de capteur indiquant l'état du produit de stomie provenant du dispositif de capteur,
dans lequel l'un ou les plusieurs dispositifs auxiliaires comportent au moins un parmi un dispositif de surveillance (32), un dispositif portable(34), un dispositif électronique grand public (36) et un dispositif serveur (38),
dans lequel l'un ou les plusieurs parmi les dispositifs auxiliaires sont configurés pour déterminer et/ou recevoir des informations de système de stomie, dans lequel l'un ou les plusieurs dispositifs auxiliaires sont configurés pour déterminer des informations caractéristiques d'un produit de stomie sur la base des informations de système de stomie, et **caractérisé en ce que**
l'un ou les plusieurs dispositifs auxiliaires sont configurés pour agréger et stocker les informations de système de stomie et/ou les informations caractéristiques provenant d'une pluralité d'utilisateurs et/ou de produits de stomie.

2. Système (10) de détection de fuites de stomie selon la revendication 1, dans lequel le dispositif de capteur (20) est un dispositif de détection de fuites configuré pour détecter une résistance électrique.

3. Système (10) de détection de fuites de stomie selon l'une quelconque des revendications 1 et 2, dans lequel l'un ou les plusieurs dispositifs auxiliaires (30) comportent le dispositif portable(34) connecté de manière fonctionnelle au dispositif de capteur et un dispositif électronique grand public (36) connecté de manière communicable au dispositif portable par l'intermédiaire d'une connexion sans fil.

4. Système (10) de détection de fuites de stomie selon l'une quelconque des revendications 1 et 2, dans lequel l'un ou les plusieurs dispositifs auxiliaires (30) comportent un dispositif de surveillance (32) couplé de manière fonctionnelle au dispositif de détection (20), un dispositif portable(34) couplé de manière à pouvoir communiquer avec le dispositif de surveillance, et un dispositif électronique grand public (36) connecté de manière à pouvoir communiquer avec le dispositif portable par le biais d'une connexion sans fil.

5. Système (10) de détection de fuites de stomie selon l'une quelconque des revendications 1 à 4, dans lequel l'au moins un dispositif auxiliaire (30) comporte un dispositif serveur (38) couplé de manière à pouvoir communiquer avec l'un ou les plusieurs parmi le dispositif portable (34) et le dispositif électronique grand public (36).

6. Système (10) de détection de fuites de stomie selon l'une quelconque des revendications 1 à 5, comprenant en outre un produit de stomie (100), dans lequel le dispositif de capteur (20) est connecté de manière fonctionnelle au produit de stomie (100) .

7. Procédé (400) d'analyse d'un système de détection de fuites de stomie, comprenant :
la réception d'informations de système de stomie relatives à un produit de stomie, dans lequel les informations de système de stomie sont des informations relatives à des facteurs affectant les performances du produit de stomie ;
la détermination d'informations caractéristiques sur la base des informations de système de stomie ;
**caractérisé en ce que** le procédé comprend l'agrégation et le stockage des informations de système de stomie et/ou des informations caractéristiques provenant d'une pluralité d'utilisateurs et/ou de produits de stomie.

8. Procédé (400) selon la revendication 7, comprenant en outre la fourniture d'une notification sur la base des informations caractéristiques.

9. Procédé (400) selon l'une quelconque des revendications 7 et 8, dans lequel les informations caractéristiques comportent au moins une parmi des informations de consommation de produit, des informations de fuite, des informations d'inventaire, des informations de santé de stomie, des informations de remplacement, des informations de recommandation et des informations prédictives.
